(19) Europäisches Patentamt
European Patent Office
Office européen des brevets

(11) Veröffentlichungsnummer : **0 283 648 B1**

(12) # EUROPÄISCHE PATENTSCHRIFT

(45) Veröffentlichungstag der Patentschrift : **05.06.91 Patentblatt 91/23**

(51) Int. Cl.$^5$ : **C07D 265/30**

(21) Anmeldenummer : **88100717.3**

(22) Anmeldetag : **20.01.88**

(54) **Verfahren zur Herstellung von cis-2,6-Dimethylmorpholin.**

Die Akte enthält technische Angaben, die nach dem Eingang der Anmeldung eingereicht wurden und die nicht in dieser Patentschrift enthalten sind.

(30) Priorität : **19.03.87 DE 3708931**

(43) Veröffentlichungstag der Anmeldung : **28.09.88 Patentblatt 88/39**

(45) Bekanntmachung des Hinweises auf die Patenterteilung : **05.06.91 Patentblatt 91/23**

(84) Benannte Vertragsstaaten : **BE CH DE FR GB IT LI NL**

(56) Entgegenhaltungen :
**DE-A- 2 830 998**
**DE-A- 2 938 698**
**DE-A- 3 322 939**
**L.F. & M.FIESER: "Reagents for organic synthesis", John Wiley & Sons, New York (US), 1967; p. 156**

(73) Patentinhaber : **HÜLS AKTIENGESELLSCHAFT**
**Patentabteilung / PB 15 - Postfach 13 20**
**W-4370 Marl 1 (DE)**

(72) Erfinder : **Borsdorff, Horst Wolfram, Dr.**
**Ammerweg 10**
**W-4200 Oberhausen (DE)**
Erfinder : **Broschinski, Lothar, Dr.**
**Lüenbrink 18**
**W-4760 Werl (DE)**
Erfinder : **Disteldorf, Josef, Dr.**
**Dahlbrede 47**
**W-4370 Marl (DE)**
Erfinder : **Hübel, Werner, Dr.**
**Keplerstr. 13**
**W-4350 Recklinghausen (DE)**
Erfinder : **Rindtorff, Klaus, Dr.**
**Keplerstr. 13**
**W-4350 Recklinghausen (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die Erfindung betrifft ein Verfahren zur Herstellung von cis-2,6-Dimethylmorpholin durch Isomerisierung von trans-2,6-Dimethylmorpholin.

Bei der Synthese von Dimethylmorpholin durch Wasserabspaltung aus Diisopropanolamin in Gegenwart saurer Katalysatoren wie beispielsweise Schwefelsäure (US-PS 3 083 202, EP-PS 0 094 565, Houben-Weyl, Bd. 6/4, Seiten 510-520) entstehen stets cis/trans-Isomerengemische. Da vielfach das cis-Isomere bevorzugt wird, beispielsweise wegen besserer Wirksamkeit von daraus erzeugten Pflanzenschutzmitteln oder Vulkanisationsbeschleunigern (DE-OSS 26 56 747, 27 52 096, 27 52 135 und US-PS 3 083 202), ist man bestrebt, das trans-2,6-Dimethylmorpholin der Formel I

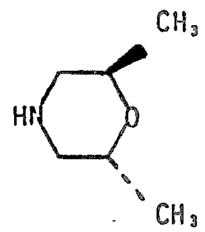

(I)

in das cis-2,6-Dimethylmorpholin der Formel (II)

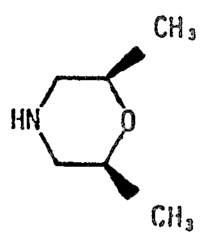

(II)

zu überführen.

Es ist bekannt, daß es auf verschiedenen Wegen gelingt, in einem Gemisch aus cis- und trans-2,6-Dimethylmorpholin den Anteil des cis-Isomeren zu erhöhen. Eine solche Methode ist die Isomerisierung des 2,6-Dimethylmorpholin-Isomerengemisches in überschüssiger konzentrierter oder rauchender Schwefelsäure bei 180 bis 220°C (US-PS 3 083 202). Auch durch eine geschicktere Prozeßführung kann der Anteil des cis-Isomeren bei Temperaturen von nur 150 bis 190°C gesteigert werden, aber ebenfalls nur bei deutlichen molaren Überschüssen an Schwefelsäure (EP-PS 0 094 565).

Nachteil beider Methoden ist außer einer mit steigendem Isomerisierungsgrad absinkenden Ausbeute bzw. erhöhten Nebenproduktbildung der Zwangsanfall von Alkalisulfaten, der sich durch Neutralisation der Morpholinbase bildet.

Demgegenüber sind verschiedene katalytische Methoden zur Isomerisierung an metallischen Hydrierkatalysatoren in Gegenwart von Wasserstoff weitgehend frei von Neben- und Abfallprodukten. So wird bei Einsatz von einem oder mehreren Metallen der Gruppen VIII oder I b des Periodensystems der Elemente bei Temperaturen von insbesondere 150 bis 250°C im günstigsten Falle ein Isomerisierungsgrad erreicht, der dem in der EP-PS 0 094 565 angegebenen Isomerenverhältnis von etwa 88% cis-Verbindung zu etwa 12 % trans-Verbindung nach Einstellung des thermodynamischen Gleichgewichtes entspricht. Nach dem Verfahren der EP-PS 0 007 520 wurden in den Beispielen 7 und 8 an einem $Pd/Pr_2O_3$-Katalysator bei Temperaturen von 230 und 250°C Umsätze von 76,7 und 86,2% bei Selektivitäten von 92,3 und 90,9% nach Destillation erreicht. Dabei wurden stündlich 60 Teile trans-2,6-Dimethylmorpholin zusammen mit 10 000 Raumteilen $H_2$ kontinuierlich über 500 Raumteilen Katalysator durchgesetzt. Palladium ist in seiner katalytischen Wirksamkeit den anderen beanspruchten Metallen überlegen.

Im weiteren wird die katalytische Isomerisierung an Metallkatalysatoren aus Pd, Zn, Cd und Mn in der EP-PS 0 026 367 und aus Pt, Ru oder Rh in der EP-A 0 129 904 beschrieben, wobei gemäß Beispiel 1 (Katalysator: Pd, Zn und Cd) ein Umsatz von 74,5% bei einer Selektivität von 94,6% und gemäß Beispiel 1a (Katalysator: Pt) ein Umsatz von 81,9% bei einer Selektivität von 97,2% nach Destillation erreicht werden.

Hierbei werden stündlich 100 Teile trans-2,6-Dimethylmorpholin mit 300 000 Raumteilen $H_2$ an 1 000 Raumteilen Katalysator bzw. 0,1 Teile trans-2,6-Dimethylmorpholin mit 100 Raumteilen $H_2$ an 1 Raumteil Kata-

lysator umgesetzt (Umsatz und Selektivität wurden aus den Literaturdaten entsprechend den Definitionen aus Ullmanns Encyklopädie der Technischen Chemie, 4. Auflage, Band 13, Seite 550, berechnet).

Nachteilig ist bei Einsatz der Isomerisierungskatalysatoren gemäß EP-PS 0 007 520 und EP-PS 0 026 367 ihr rascher Aktivitätsabfall infolge des herstellungsbedingten Gehaltes an Schwefelverbindungen im 2,6-Dimethylmorpholin technischer Qualität. Eine Ausnahme bilden hier lediglich die zuletzt genannten Edelmetallkatalysatoren auf Basis Pt, Ru oder Rh. Ebenfalls nachteilig sind bei allen drei obengenannten Verfahren die hohen Preise der Edelmetallkatalysatoren.

Der Erfindung liegt die Aufgabe zugrunde, ein wirtschaftlicheres Verfahren zur Isomerisierung von schwefelhaltigem trans-2,6-Dimethylmorpholin zu finden, das zudem noch höhere Umsatzraten aufweist.

Diese Aufgabe wird erfindungsgemäß dadurch gelöst, daß man trans-2,6-Dimethylmorpholin in Gegenwart von Wasserstoff und einem vorher mittels Wasserstoff aktivierten Kupferchromit-Katalysator bei Temperaturen von 180 bis 300°C und Drücken von 1 bis 500 bar (abs.) isomerisiert, insbesondere bei Temperaturen von 220 bis 280°C und bei Drücken von 1 bis 300 bar (abs.).

Es ist überraschend, daß mit diesem in wirtschaftlicher Weise herstellbaren Katalysatortyp noch bessere Ergebnisse bezüglich der Umsatzrate als mit den obengenannten Edelmetallkatalysatoren erzielt werden können. Die Selektivität der Isomerisierungsreaktion erreicht teilweise fast Werte von 100%. Selbst bei schwefelhaltigem Einsatzprodukt waren gute Katalysatorstandzeiten möglich, wobei der Schwefelgehalt des eingesetzten trans-2,6-Dimethylmorpholins in der Größenordnung von 2 bis 10 Gewichts-ppm lag.

Das erfindungsgemäße Verfahren arbeitet in Gegenwart von Wasserstoff und bevorzugt in der Gasphase. Bei höheren Drücken erfolgt die Umsetzung dagegen in der Flüssigphase im Riesel- oder Sumpfverfahren. Dabei kann auch unter Anwendung inerter Lösungsmittel wie Alkane, Ether oder Glykole gearbeitet werden.

Der Katalysator ist ein Kupferchromit, der vorzugsweise noch zusätzlich Bariumoxid und/oder Mangandioxid enthält. Vor Einsatz zur Isomerisierung muß der Katalysator durch eine reduktive Behandlung mittels Wasserstoff aktiviert werden, wobei sorgfältig eine vollständige Reduktion bis zum metallischen Kupfer vermieden werden muß. Diese Aktivierung erfolgt, indem das Katalysatorbett unter einem Stickstoffstrom auf 130°C aufgeheizt wird und dann schrittweise ein Austausch von Stickstoff gegen Wasserstoff unter Beachtung der dabei freiwerdenden Reaktionswärme mit Begrenzung der Kontaktbett-Temperatur auf maximal 160°C vorgenommen wird. Es kommt dabei zu einer Teilreduktion bis zur Stufe des einwertigen Kupfers, wobei sich gleichzeitig die entsprechende Menge an Reaktionswasser bildet. Jede Überreduzierung des Katalysators führt zu einer nachhaltigen Verschlechterung des Isomerisierungsgrades.

Außer CuO, $Cr_2O_3$ und bevorzugt noch zusätzlich BaO und/oder $MnO_2$ enthält der Katalysator ein zur Formgebung notwendiges alkalisches Bindemittel. Die Gehalte an BaO und/oder $MnO_2$ dienen zur Unterdrückung einer zu weitgehenden Reduktion. Denn während ein oxidischer Kupferchromit, besonders bei erwähnter Stabilisierung, als Hydrierkatalysator Verwendung findet, ändert ein bis zum metallischen Kupfer reduzierter Kupferchromit-Katalysator seine Charakteristik in einen Dehydrogenierungskatalysator. Typische Handelsprodukte solcher mit BaO dotierten Kupferchromit-Katalysatoren sind beispielsweise Mallinckrodt E 406 TU cored und Harshaw Cu 1107 T 1/8", deren Gehalt an CuO sich auf 42 bis 33% beläuft.

Der Katalysator kann in allen gebräuchlichen Formen, die zum Aufbau eines Festbettes geeignet sind, eingesetzt werden, wie zum Beispiel auch in Form von Tabletten, Pellets, Ringen und Extrudaten. Ebenfalls kann Kupferchromit auf einem inerten Trägermaterial verwendet werden.

Die Erfindung wird durch die folgenden Beispiele erläutert :

Legende für die nachstehenden Tabellen :

| Verfahrensparameter/ Versuchsergebnis | Abkürzung in der Tabelle |
|---|---|
| Verfahrensdruck in bar (abs.) | Druck [bar] |
| Verfahrenstemperatur in °C | Temp. [°C] |
| Einsatzstoffgemisch aus cis-/trans-2,6-Dimethylmorpholin in l/h oder ml/h | Einsatz [l/h] oder [ml/h] |
| Wasserstoffstrom in $Nm^3$/h oder l/h | $H_2$-Strom [$Nm^3$/h] oder [l/h] |
| Gehalt an cis-2,6-Dimethylmorpholin in Gew.-% im Reaktionsprodukt laut gaschromatographischer Analyse | GC: 2,6-DMM [Gew.-%] cis- |
| Gehalt an trans-2,6-Dimethylmorpholin in Gew.-% im Reaktionsprodukt laut gaschromatographischer Analyse | GC: 2,6-DMM [Gew.-%] trans- |
| Umsatz in % | Umsatz % |
| Selektivität in % | Selektivität % |

Die Werte für Umsatz und Selektivität wurden einheitlich nach den entsprechenden Definitionen aus Ullmanns Encyklopädie der Technischen Chemie, 4. Auflage, Band 13, Seite 550, berechnet:

Umsatz U

$$U = \frac{\text{umgesetzte Mole i}}{\text{eingesetzte Mole i}} \cdot 100 \left[\frac{\text{mol} \cdot \%}{\text{mol}}\right]$$

Selektivität S

$$S = \frac{\text{in j umgewandelte Mole i}}{\text{umgesetzte Mole i}} \cdot 100 \left[\frac{\text{mol} \cdot \%}{\text{mol}}\right]$$

Beispiel 1

In einen 1 l-Schachtofen werden 500 ml eines Katalysators vom Typ Mallinckrodt E 406 TU cored, der 42 Gew.-% CuO, 40% $Cr_2O_3$, 8% BaO und 10% Bindemittel enthält, eingefüllt und auf die gewünschte Temperatur erhitzt. Das eingesetzte 2,6-Dimethylmorpholin (5,7 Gew.-% cis- und 91,9% trans-Verbindung) wird zusammen mit dem Wasserstoffstrom je nach dem Systemdruck entweder flüssig im Rieselverfahren oder durch Verdampfen auf der über dem Kontakt angebrachten Füllkörperschicht in der Gasphase durchgesetzt.

Die Ergebnisse sind in Tabelle 1 aufgelistet.

Tabelle 1:

| Zeile | Temp. [°C] | Druck [bar] | $H_2$-Strom [l/h] | Einsatz [ml/h] | GC: 2,6-DMM [Gew.-%] cis- | trans- | Umsatz % | Selektivitat % |
|---|---|---|---|---|---|---|---|---|
| 1 | 220 | 3 | 50 | 100 | 84,27 | 10,84 | 88,15 | 97,82 |
| 2 | 240 | 3 | 50 | 100 | 85,11 | 8,95 | 90,26 | 95,70 |
| 3 | 240 | 21 | 50 | 100 | 84,18 | 12,28 | 86,64 | 98,53 |
| 4 | 240 | 101 | 50 | 100 | 73,56 | 21,47 | 76,65 | 96,30 |
| 5 | 240 | 101 | 500 | 100 | 72,45 | 23,15 | 74,82 | 97,05 |
| 6 | 260 | 16 | 500 | 250 | 80,42 | 11,72 | 87,19 | 94,07 |
| 7 | 260 | 21 | 500 | 250 | 81,48 | 11,14 | 87,89 | 93,79 |
| 8 | 260 | 21 | 5 000 | 250 | 81,44 | 14,08 | 84,69 | 97,28 |
| 9 | 260 | 101 | 500 | 250 | 73,63 | 20,32 | 77,90 | 94,85 |
| 10 | 260 | 261 | 500 | 100 | 67,69 | 22,77 | 75,11 | 90,67 |

Es zeigt sich, daß die Isomerisierung vorteilhafter in der Gasphase (3 bar) als in der Flüssigphase (16, 21, 101, 261 bar) abläuft. Eine Steigerung des Wasserstoffdurchsatzes wirkt umsatzmindernd und selektivitätsfördernd, bleibt aber letztlich in der Ausbeute ohne signifikante Auswirkung.

Bei einer Dauerbeanspruchung von über 500 Betriebsstunden war kein Aktivitätsabfall des Katalysators zu verzeichnen.

Beispiel 2

Mit der in Beispiel 1 beschriebenen Versuchsanordnung wurde ein Katalysator vom Typ Harshaw Cu 1107 T 1/8", der 33 Gew.-% CuO, 38% $Cr_2O_3$, 9% BaO und 20% Bindemittel enthält, getestet. Das Einsatzprodukt enthält 7,0 Gew.-% cis- und 88,0% trans-2,6-Dimethylmorpholin.

Die Ergebnisse sind in Tabelle 2 zusammengestellt.

Tabelle 2:

| Zeile | Temp. [°C] | Druck [bar] | $H_2$-Strom [l/h] | Einsatz [ml/h] | GC: 2,6-DMM [Gew.-%] cis- | trans- | Umsatz % | Selektivität % |
|---|---|---|---|---|---|---|---|---|
| 1 | 240 | 1,25 | 50 | 150 | 73,5 | 17,0 | 80,7 | 93,7 |
| 2 | 240 | 3 | 50 | 150 | 79,1 | 11,7 | 86,7 | 94,5 |
| 3 | 240 | 4 | 50 | 150 | 80,7 | 11,1 | 87,4 | 95,8 |
| 4 | 240 | 6 | 50 | 150 | 78,4 | 14,3 | 83,8 | 96,9 |
| 5 | 240 | 11 | 50 | 150 | 77,7 | 15,4 | 82,5 | 97,4 |
| 6 | 240 | 21 | 50 | 150 | 72,6 | 21,2 | 75,9 | 98,2 |

Bis zu einem Druck von 4 bar abs. nimmt die Umsatzrate zu, bei weiter ansteigendem Druck fällt sie dann ab. Die Selektivität steigt mit höheren Drücken stetig an.

Bei einer Dauerbeanspruchung von über 400 Betriebsstunden war kein Nachlassen der Wirksamkeit des Katalysators zu beobachten.

Beispiel 3

Mit der in Beispiel 1 beschriebenen Versuchsanordnung wurde die Isomerisierung von trans-2,6-Dimethylmorpholin mit einem $MnO_2$-dotierten Katalysator vom Typ Harshaw Cu 1932 T 1/8", der 35,7 Gew.-% CuO, 30,7% $Cr_2O_3$, 2,72% $MnO_2$ und 30,88% Bindemittel enthält, durchgeführt. Als Einsatzprodukt wird ein Gemisch aus 5,64 Gew.-% cis- und 91,95% trans-Verbindung verwendet.

Die Ergebnisse sind in Tabelle 3 aufgeführt.

Tabelle 3:

| Zeile | Temp. [°C] | Druck [bar] | $H_2$-Strom [l/h] | Einsatz [ml/h] | GC: 2,6-DMM [Gew.-%] cis- | trans- | Umsatz % | Selektivität % |
|---|---|---|---|---|---|---|---|---|
| 1 | 240 | 3 | 50 | 50 | 82,06 | 8,38 | 90,88 | 91,45 |
| 2 | 240 | 3 | 50 | 250 | 77,55 | 15,70 | 82,93 | 94,31 |
| 3 | 220 | 3 | 50 | 100 | 84,76 | 12,08 | 86,87 | 99,06 |

Bei einer Dauerbeanspruchung von über 200 Betriebsstunden war kein Aktivitätsabfall des Katalysators zu verzeichnen.

Beispiel 4

Es wurde mit der in Beispiel 1 beschriebenen Versuchsanordnung ein unstabilisierter Kupferchromit-Katalysator vom Typ Harshaw Cu 1808 T 1/8", der 42,5 Gew.-% CuO, 38,75% $Cr_2O_3$ und 18,75% Bindemittel enthält, getestet. Das Einsatzprodukt hat dieselbe Zusammensetzung wie in Beispiel 3.

Die Ergebnisse sind in Tabelle 4 aufgelistet.

Tabelle 4:

| Zeile | Temp. [°C] | Druck [bar] | $H_2$-Strom [l/h] | Einsatz [ml/h] | GC: 2,6-DMM [Gew.-%] cis- | trans- | Umsatz % | Selektivität % |
|---|---|---|---|---|---|---|---|---|
| 1 | 240 | 3 | 50 | 100 | 71,71 | 25,65 | 72,10 | 99,65 |
| 2 | 260 | 3 | 50 | 100 | 74,59 | 19,85 | 78,41 | 95,62 |

Dieser weder mit BaO noch mit $MnO_2$ dotierte Katalysator erreicht nicht ganz so hohe Umsatzraten wie die Katalysatoren in den vorhergegangenen Beispielen.

Beispiel 5 (Vergleichsversuch)

Mit der in Beispiel 1 beschriebenen Versuchsanordnung wurde zum Vergleich ein reiner Kupferkatalysator vom Typ Harshaw Cu 2501 G, der 6 Gew.-% Cu als $CuCO_3$ auf einem Silica-Träger enthält, getestet. Das Einsatzprodukt hat dieselbe Zusammensetzung wie in Beispiel 3.

Die Ergebnisse sind in Tabelle 5 wiedergegeben :

Tabelle 5:

| Zeile | Temp. [°C] | Druck [bar] | $H_2$-Strom [l/h] | Einsatz [ml/h] | GC: 2,6-DMM [Gew.-%] cis- | trans- | Umsatz % | Selektivität % |
|---|---|---|---|---|---|---|---|---|
| 1 | 240 | 3 | 50 | 100 | 34,80 | 61,33 | 33,3 | 95,2 |
| 2 | 260 | 3 | 50 | 100 | 30,84 | 67,92 | 29,7 | 91,7 |

Es ist deutlich zu erkennen, daß die Umsatzraten bei diesem Katalysator weit hinter denen der Kupferchromit-Katalysatoren zurückbleiben.

Beispiel 6

Ein Röhrenbündelreaktor mit 108 Reaktionsrohren von jeweils 4 cm Durchmesser und 240 cm Länge wird mit insgesamt 432,8 kg = 325 l eines Katalysators vom Typ Mallinckrodt E 406 TU cored, der 42 Gew.-% CuO, 40% $Cr_2O_3$, 8% BaO und 10% Bindemittel enthält, gefüllt. Durch Durchleiten eines Stickstoffstromes von 30 $Nm^3/h$ bei 150°C Reaktionstemperatur und allmählichem Austausch von Stickstoff gegen Wasserstoff wird der Kontakt drucklos aktiviert, danach langsam auf 2 bar Überdruck gebracht und die Systemtemperatur auf 240 °C eingestellt.

Nach Einstellung der gewünschten Reaktionsbedingungen werden stündlich 115 l = 107 kg 2,6-Dimethylmorpholin in einem Verdampfer in die Gasphase überführt und anschließend zusammen mit dem vorgeheizten Wasserstoffstrom in einem Überhitzer auf die im Reaktor herrschende Temperatur gebracht und durch das Katalysatorbett geleitet. Das den Reaktor verlassende Reaktionsgemisch wird über einen Kühler und Abscheider in 2,6-Dimethylmorpholin und Wasserstoff getrennt und der Wasserstoffüberschuß wieder dem Reaktor zugeführt.

Das anfallende 2,6-Dimethylmorpholin zeigt gegenüber dem eingesetzten 2,6-Dimethylmorpholin eine hohe Isomerisierungsrate bei guter Selektivität. In 720 Betriebsstunden wurden ohne Anzeichen einer nachlassenden Katalysatoraktivität in Abhängigkeit vom Wasserstoff-Durchsatz bei sonst konstanten Bedingungen mit einem Einsatzprodukt, dessen Gehalt an 2,6-Dimethylmorpholin sich auf 12,88% cis- und 82,66% trans-Verbindung belief, folgende Resultate im Austrittsprodukt erzielt:

Tabelle 6:

| Zeile | Temp. [°C] | Druck [bar] | $H_2$-Strom [$Nm_3$/h] | Einsatz [l/h] | GC: 2,6-DMM [Gew.-%] cis- | trans- | Umsatz % | Selektivität % |
|---|---|---|---|---|---|---|---|---|
| 1 | 240 | 3 | 40 | 115 | 84,90 | 7,36 | 91,1 | 95,6 |
| 2 | 240 | 3 | 30 | 115 | 84,24 | 7,61 | 90,8 | 95,0 |
| 3 | 240 | 3 | 20 | 115 | 84,21 | 7,11 | 91,4 | 94,4 |

Diese Ergebnisse zeigen, daß gute Umsatz- und Selektivitätswerte bei gleichzeitig hohen Kontaktbelastungen und -standzeiten erreichbar sind.

## Ansprüche

1. Verfahren zur Herstellung von cis-2,6-Dimethylmorpholin durch Isomerisierung von trans-2,6-Dimethylmorpholin, dadurch gekennzeichnet, daß man trans-2,6-Dimethylmorpholin in Gegenwart von Wasserstoff und einem vorher mittels Wasserstoff aktivierten Kupferchromit-Katalysator bei Temperaturen von 180 bis 300°C und Drücken von 1 bis 500 bar (abs.) isomerisiert.

2. Verfahren nach Anspruch 1, dadurch gekennzeichnet, daß man in Gegenwart eines Kupferchromit-Katalysators isomerisiert, der zusätzlich Bariumoxid und/oder Mangandioxid enthält.

3. Verfahren nach den Ansprüchen 1 und 2, dadurch gekennzeichnet, daß man bei Temperaturen von 220 bis 280°C isomerisiert.

4. Verfahren nach den Ansprüchen 1 bis 3, dadurch gekennzeichnet, daß man bei Drücken von 1 bis 300 bar (abs.) isomerisiert.

## Claims

1. A process for the preparation of cis-2,6-dimethyl morpholine by the isomerization of trans-2,6-dimethyl morpholine, characterized in that trans-2,6-dimethyl morpholine is isomerized in the presence of hydrogen and a copper chromite catalyst previously activated by means of hydrogen, at temperatures of 180 to 300°C and pressures of 1 to 500 bar (abs).

2. A process according to claim 1, characterized in that isomerization is performed in the presence of a copper chromite catalyst which also contains barium oxide and/or manganese dioxide.

3. A process according to claims 1 and 2, characterized in that isomerization is performed at temperatures of 220 to 280°C.

4. A process according to claims 1 to 3, characterized in that isomerization is performed at pressures of 1 to 300 bar (abs).

**Revendications**

1. Procédé de fabrication de cis-2,6-diméthylmorpholine par isomérisation de trans-2,6-diméthylmorpholine, caractérisé en ce que l'on isomérise la trans-2,6-diméthylmorpholine en présence d'hydrogène et d'un catalyseur au chromite de cuivre préalablement activé à l'aide d'hydrogène à des températures allant de 180 à 300°C et à des pressions de 1 à 500 bars (abs).

2. Procédé selon la revendication 1, caractérisé en ce que l'on effectue l'isomérisation en présence d'un catalyseur au chromite de cuivre qui contient en addition de l'oxyde de baryum et/ou du dioxyde de manganèse.

3. Procédé selon les revendications 1 et 2, caractérisé en ce que l'on effectue l'isomérisation àdes températures de 220 à 280°C,

4. Procédé selon les revendications 1 à 3, caractérisé en ce que l'on effectue l'isomérisation à des pressions allant de 1 à 300 bars (abs.).